# FASCICULE DE BREVET EUROPEEN

(11) **EP 1 029 239 B1**
(45) Date de publication et mention de la délivrance du brevet: **24.03.2004**
(21) Numéro de dépôt: 99906114.6
(22) Date de dépôt: 11.01.1999
(51) Int. Cl.: G01N 33/00

(54) **PROCEDE ET DISPOSITIF DE DETECTION DE SUBSTANCES NOCIVES**
VERFAHREN UND VORRICHTUNG ZUM NACHWEIS VON SCHÄDLICHEN SUBSTANZEN
METHOD AND DEVICE FOR DETECTING HARMFUL SUBSTANCES

(30) Priorité: 15.01.1998 LU 90196
(43) Date de publication de la demande: 23.08.2000
(73) Titulaire: Health and Safety Systems Holding S.A., 8156 Bridel (LU)
(72) Inventeur: GOOSSENS, Renier, J., L., C., L-8156 Bridel (LU); SCHINKEL, Robert, NL-2396 JD Koudekerk a/d Rijn (NL)
(74) Mandataire: Kihn, Pierre Emile Joseph
(86) Numéro de dépôt international: PCT/EP1999/000112
(87) Numéro de publication internationale: WO 1999/036771

(56) Documents cités:
- EP-A- 0 527 307
- GB-A- 2 233 088
- "MESURES DE POLLUTION" MESURES REGULATION AUTOMATISME, vol. 48, no. 6, 18 avril 1983, page 7/8, 10/11, 14/1 XP002035169
- PATENT ABSTRACTS OF JAPAN vol. 8, no. 84 (E-239) & JP 59 004354 A (FUJITSU KK), 11 janvier 1984

## Description

### Introduction

La présente invention concerne un dispositif de détection de substances nocives.

Afin de prévenir des maladies professionnels dues à l'exposition à des substances nocives. Il est important de bien contrôler l'exposition des travailleurs à ces substances nocives afin de pouvoir intervenir en cas de dépassement des doses maximales autorisées.

Par substances nocives on entend non seulement des substances chimiques mais également des rayonnements divers comme les rayons U.V., les rayons X, le bruit, la lumière UV, etc.

Comme certaines maladies ayant à l'origine une exposition à ces substances dangereuses peuvent se déclarer seulement après des années voir des décennies, il est important de suivre l'évolution de l'exposition de chaque individu à chaque substance identifiée comme nocive.

Le document "American Industrial Hygien Association Journal", Vol. 48 No 2. Février 1987 page A119 décrit un dispositif portable de surveillance de la concentration d'isocyanates dans l'air. On annonce que dans le futur, ce système pourra surveiller d'autres gaz dans l'atmosphère comme p.ex. le phosgène, les hydrazines dès que de nouveaux détecteurs à ruban en papier adéquats sont disponibles. Le dispositif portable permet de mesurer et d'enregistrer sur une période d'environ huit heures un profil de concentration et déclenche une alarme lorsque la concentration dépasse un certain seuil. Les données recueillies par le dispositif portable sont peuvent être analysées et revues sur un ordinateur de type IBM moyennant une interface et un logiciel livré.

EP-A-0 527 307 publié le 17.02.93, décrit un système de détection et de management de la pollution urbaine. Le système comprend des stations de mesures fixes et des stations de mesure mobiles permettant de détecter des polluants atmosphériques, un centre permettant de stocker, de retirer, de gérer et de traiter les données en provenance des stations de mesure en vue de faire des prévisions et de distribuer ces données et prévisions en vue de prendre des actions visant à diminuer la pollution atmosphérique.

### Objet de l'invention

L'objet de la présente invention est de proposer un procédé et un dispositif de détection de substances nocives permettant de connaître à tout moment les doses aux quelles un travailleur déterminé a été exposé.

### Description générale de l'invention

Conformément à l'invention, cet objectif est atteint par un dispositif de détection de substances nocives comprenant :
a) un poste portable comprenant un dispositif permettant de détecter au moins une substance nocive et d'en mesurer la concentration et un dispositif de sauvegarde des données sur l'identité et les concentrations en substances nocives, recueillies par le poste portable,
b) une station fixe permettant de décharger et de stocker les données recueillies par le poste portable,
c) un ordinateur central pouvant être raccordé a la station fixe permettant de décharger les données de la station fixe et d'évaluer les données recueillies;
   caractérisé en ce
c) qu'il comprend également un crypteur de données permettant de cryptographier les données recueillies par le poste portable,
d) et en ce que le dispositif de détection comprend un module enclenchable lorsque le poste portable est utilisé par une personne portant des vêtements de protection.

Un des avantages de système est que les données recueillies sont enregistrées dans un ordinateur central qui peut faire l'évaluation des données d'un grand nombre de stations fixes et donc de postes mobiles.

D'autres modes de réalisations préférés sont décrits dans les revendications dépendantes.

### Description à l'aide des Figures

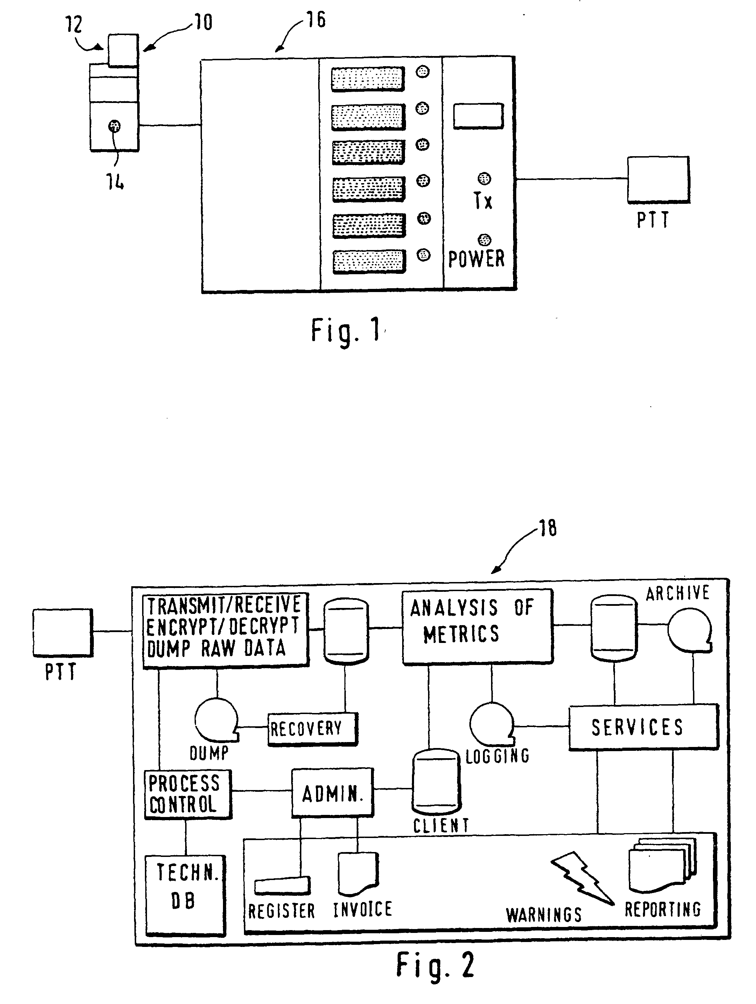

D'autres particularités et caractéristiques de l'invention ressortiront de la description détaillée de quelques modes de réalisation avantageux présentés ci-dessous, à titre d'illustration, en référence aux dessins annexés. Celles ci montrent
- Fig.1:: le poste mobile et la station fixe de façon schématique,
- Fig.2:: un organigramme présentant le fonctionnement de l'ordinateur permettant d'évaluer les données recueillies par les stations mobiles.
Le poste mobile 10 représenté à la fig. 1 est un dispositif dont la taille réduite permet de le porter dans ou sur la poche pectorale d'un vêtement.
Le poste mobile comprend un dispositif 12 permettant de détecter et de mesurer la concentration d'au moins une substance nocive, une source d'énergie (non représentée), un dispositif de sauvegarde des données sur l'identité et les concentrations en substances nocives (non représenté), recueillies par le poste mobile 10, un dispositif d'alarme 14 visuel et/ou auditif qui est déclenché lorsque la concentration en substances nocives dépasse un seuil prédéterminé et un dispositif de connexion (non représenté) permettant de le connecter à une station fixe 16 afin de décharger les données recueillies sur la station fixe 16.
Le poste mobile 10 surveille en permanence ou à des intervalles réguliers l'environnement du travailleur en ce qui concerne les substances nocives qu'il est capable de détecter et de mesurer.
Le poste mobile 10 permet donc de surveiller et d'enregistrer l'exposition à une substance nocive du porteur.
Chaque poste mobile 10 est pourvu en outre d'une horloge interne permettant d'enregistrer les concentrations en substances nocives mesurées au cours du temps ensemble avec les données en relation avec le temps auquel elles ont été mesurées. On pourra donc, le cas échéant, compiler un profil des concentrations auxquelles le porteur du poste mobile 10 a été exposé en fonction du temps.
Le poste mobile 10 comprend en outre un module enclenchable lorsqu'il est utilisé par une personne portant des vêtements de protection. Il est important de souligner que le poste mobile 10 doit être porté sur les vêtements resp. sur les vêtements de protection. Dans le cas où la personne utilisant un poste mobile 10 porte des vêtements de protection, ce module s'enclenche et les concentrations mesurées sont adaptées à l'aide d'un algorithme en fonction du type de vêtements portés.
A la fin de chaque journée de travail le poste mobile est placé sur la station fixe 16 et les données recueillies par le poste mobile 10 sont déchargées sur la station fixe puis stockées ensemble avec l'identification du poste mobile 10 dont elles proviennent. Après vérification des données transmises, celles-ci sont effacées de la mémoire du poste mobile 10.
Dans une application typique de travailleurs effectuant des travaux de peintures au pistolet, le poste mobile est équipé de détecteurs capables de détecter le polyuréthane, le chrome et le plomb.
Pour d'autres applications, le poste mobile peut être équipée de détecteurs de bruits, de radiations, de lumière etc..
La station fixe 16 permet également de recharger les accumulateurs du poste mobile, de reprogrammer les concentrations déclenchant un alarme, de synchroniser l'horloge interne du poste mobile, de reprogrammer le poste mobile dans le cas de changement d'un dispositif permettant de détecter et de mesurer la concentration en substances nocives.
La station fixe 16 peut être connectée à un ordinateur central 18. Ceci peut se faire au moyen d'une ligne téléphonique et d'un modem. Les données stockées dans la station fixe sont cryptées, transmises à l'ordinateur central 18 puis effacées de la mémoire de la station fixe 16 après vérification de l'intégrité des données transmises.
La station fixe 16 peut être reprogrammée uniquement à partir de l'ordinateur central 18. La station fixe 16 et les postes mobiles 10 sont des boites noires en ce sens que les données du poste portable ne peuvent être recueillies que par une station fixe 16 adéquate et que l'accès à la station fixe ne peut s'effectuer que par l'ordinateur central 18. Ceci permet d'éviter la manipulation des données au niveau du poste mobile 10 et/ou de la station fixe 16.
L'ordinateur central 18 est le point d'enregistrement dans lequel les données de tous les postes mobiles 10 sont finalement stockées, analysées et évaluées.
Par mesure de sécurité et de fiabilité du système, les données recueillies par l'ordinateur central 18 en provenance des différentes stations fixes 16 sont enregistrées séparément en deux points différents. Tous les calculs et analyses sont faits sur une seule des deux copie de données afin que l'autre copie de données soit maintenue intacte et permette de récupérer les données originaux en cas de problèmes.
Lorsque l'ordinateur central 18 détecte un nouveau poste mobile 10 ou bien si les détecteurs ont été changés, les paramètres appropriés sont mis à disposition du poste mobile concerné.
Le principe de fonctionnement de l'ordinateur central est schématisé sur l'organigramme de la fig. 2

## Revendications

1. Dispositif de détection de substances nocives comprenant :
a) un poste portable comprenant un dispositif permettant de détecter au moins une substance nocive et d'en mesurer la concentration et un dispositif de sauvegarde des données sur l'identité et les concentrations en substances nocives, recueillies par le poste portable,
b) une station fixe permettant de décharger et de stocker les données recueillies par le poste portable,
c) un ordinateur central pouvant être raccordé a la station fixe permettant de décharger les données de la station fixe et d'évaluer les données recueillies;
**caractérisé en ce**
c) qu'il comprend également un crypteur de données permettant de cryptographier les données recueillies par le poste portable,
d) et en ce que le dispositif de détection comprend un module enclenchable lorsque le poste portable est utilisé par une personne portant des vêtements de protection.

2. Dispositif de détection selon la revendication 1, **caractérisé en ce que** le poste portable comprend un dispositif d'alarme qui est déclenchable lorsque la concentration mesurée en substances nocives dépasse un seuil prédéterminé.

3. Dispositif de détection selon la revendication 1 ou 2, **caractérisé en ce que** la station fixe peut recueillir les données en provenance de plusieurs postes portables différents.

4. Dispositif de détection selon l'une quelconque des revendications précédentes, **caractérisé en ce que** le poste portable peut être programmé à partir de la station fixe.

5. Dispositif de détection selon l'une quelconque des revendications précédentes, **caractérisé en ce que** la station fixe peut être programmée à partir de l'ordinateur central.

6. Dispositif de détection selon l'une quelconque des revendications précédentes, **caractérisé en ce que** le poste portable comprend une horloge interne permettant d'attribuer une mesure du temps à chaque mesure de substance nocive.

## Claims

1. Device for detecting noxious substances, comprising:
a) a portable unit comprising a device for detecting at least one noxious substance and for measuring its concentration, and a device for saving the data about the identity and the concentrations of noxious substances, which are collected by the portable unit,
b) a fixed station for downloading and storing the data collected by the portable unit,
c) a central computer which can be connected to the fixed station, to allow the downloading of the data from the fixed station and the analysing of the collected data;
**characterized in that**
c) it also comprises a data encrypter for encrypting the data collected by the portable unit,
d) and **in that** the detection device comprises a module which can be engaged when the portable unit is being used by a person wearing protective clothing.

2. Detection device according to Claim 1, **characterized in that** the portable unit comprises an alarm device, which can be triggered when the measured concentration of noxious substances exceeds a predetermined threshold.

3. Detection device according to Claim 1 or 2, **characterized in that** the fixed station can collect the data coming from a plurality of different portable units.

4. Detection device according to any one of the preceding claims, **characterized in that** the portable unit can be programmed from the fixed station.

5. Detection device according to any one of the preceding claims, **characterized in that** the fixed station can be programmed from the central computer.

6. Detection device according to any one of the preceding claims, **characterized in that** the portable unit comprises an internal clock for assigning a time record to each noxious substance measurement.

## Patentansprüche

1. Vorrichtung zum Nachweis von schädlichen Substanzen, bestehend aus
a) einem tragbaren Gerät, das eine Vorrichtung, die es ermöglicht, mindestens eine schädliche Substanz aufzuspüren und deren Konzentration zu messen, und eine Vorrichtung zur Sicherung der vom tragbaren Gerät aufgenommenen Daten bezüglich Art und Konzentrationen der schädlichen Substanzen umfasst,
b) einer festen Datenstation, die es ermöglicht, die vom tragbaren Gerät aufgenommenen Daten herunter zu laden und zu speichern,
c) einem Zentralrechner, der an die feste Datenstation angeschlossen werden kann und es ermöglicht, deren Daten herunter zu laden und die aufgenommenen Daten auszuwerten,
**dadurch gekennzeichnet, dass**
c) sie auch ein Datenverschlüsselungstool umfasst, das es ermöglicht, die vom tragbaren Gerät aufgenommenen Daten zu verschlüsseln,
d) die Aufspürvorrichtung ein Modul umfasst, das einschaltbar ist, wenn das tragbare Gerät von einer Person benutzt wird, welche Schutzkleider trägt.

2. Aufspürvorrichtung nach Anspruch 1 **dadurch gekennzeichnet, dass** das tragbare Gerät eine Alarmvorrichtung umfasst, die ausgelöst werden kann, wenn die gemessene Konzentration an schädlichen Substanzen einen vorher festgelegten Grenzwert überschreitet.

3. Aufspürvorrichtung nach Anspruch 1 oder 2 **dadurch gekennzeichnet, dass** die feste Datenstation Daten von mehreren, unterschiedlichen tragbaren Geräten aufnehmen kann.

4. Aufspürvorrichtung nach irgendeinem der vorstehenden Ansprüche **dadurch gekennzeichnet, dass** das tragbare Gerät von der festen Datenstation aus programmiert werden kann.

5. Aufspürvorrichtung nach irgendeinem der vorstehenden Ansprüche **dadurch gekennzeichnet, dass** die feste Datenstation vom Zentralrechner aus programmiert werden kann.

6. Aufspürvorrichtung nach irgendeinem der vorstehenden Ansprüche **dadurch gekennzeichnet, dass** das tragbare Gerät eine interne Uhr umfasst, die es ermöglicht, einer jeden Messung einer schädlichen Substanz eine Zeitmessung zuzuordnen.
